# EUROPÄISCHE PATENTSCHRIFT

(11) **EP 0 965 312 B1**
(45) Veröffentlichungstag und Bekanntmachung des Hinweises auf die Patenterteilung: **27.08.2003**
(21) Anmeldenummer: 99111631.0
(22) Anmeldetag: 16.06.1999
(51) Int. Cl.: A61F 2/30

(54) **Zementfrei implantierbare Endoprothese aus Metall**
Metal endoprothesis which can be implanted without cement
Endoprothèse métallique, implantable sans ciment

(30) Priorität: 17.06.1998 DE 29810818 U
(43) Veröffentlichungstag der Anmeldung: 22.12.1999
(73) Patentinhaber: ESKA Implants GmbH & Co., 23556 Lübeck (DE)
(72) Erfinder: Henssge, Ernst Joachim, 23562 Lübeck (DE); Broziat, Horst, 23554 Lübeck (DE)
(74) Vertreter: Fuchs Mehler Weiss & Fritzsche

(56) Entgegenhaltungen:
- EP-A- 0 417 034
- EP-A- 0 672 395
- WO-A-95/12369
- FR-A- 2 433 336
- US-A- 4 851 008
- US-A- 4 865 608

## Beschreibung

Die Erfindung betrifft eine zementfrei implantierbare Endoprothese aus Metall, mit mehreren an der Oberfläche eingeformten, im Querschnitt hinterschnittenen Nuten, sowie ein Verfahren zur Herstellung derartiger Endoprothesen.

Eine Prothese gemäß dem Oberbegriff von Anspruch 1 ist aus der US-A-4 851 008 bekannt.

Aus der DE 196 06 257 ist ein metallisches Formteil für ein Knochenimplantat bekannt, dessen Oberfläche mindestens abschnittsweise eine Struktur aus einer Vielzahl von Ansätzen aufweist. Die Ansätze sind stabförmig ausgebildet und besitzen an ihren freien Enden jeweils einen verbreiterten Kopf, so daß hinter den Köpfen hinterschnittene Zwischenräume vorhanden sind, in die das Knochengewebe hineinwachsen und dadurch das Implantat dauerhaft verankern kann. Die Ansätze sind in regelmäßigem Abstand voneinander oder in einer gewünschten Abstandsverteilung angeordnet, so daß das Knochengewebe nach der Implantation des Formteils mit einer vergleichsweise regelmäßigen Formstruktur konfrontiert ist, die eine homogene Festigkeit an der Grenzfläche zwischen Knochengewebe und Formteil realisiert. Diese bekannten Formteile lassen sich wegen der komplexen Form der Ansätze nur mit großem Aufwand herstellen.

Aus der US 4,623,349 ist eine Endoprothese mit einem Schaft bekannt, welcher in seiner Längserstreckung mehrere geradlinig verlaufende, hinterschnittene Nuten aufweist. Die Stege zwischen den Nuten verlaufen in Längsrichtung keilförmig, der lichte Querschnitt der Nuten nimmt zum freien, distalen Schaftende hin zu. Diese bekannte Oberflächenstruktur hat zur Folge, daß beim Implantieren des Schaftes Knochengewebe, welches im Bereich der Längsnuten vorhanden ist, zunehmend verdichtet wird, wenn der Schaft zunehmend in den Knochenhohlraum eingeführt wird und seine Endstellung allmählich erreicht. Auf diese Weise wird eine vergleichsweise hohe Primärstabilität der Endoprothese nach ihrer Implantation erreicht, die Nutenstruktur an der Schaftoberfläche ist jedoch nur mit großem Aufwand herstellbar und geht mit unerwünschten Gefügeänderungen an der Materialoberfläche einher.

Aus dem deutschen Gebrauchsmuster G 86 07 873.9 ist eine zementfrei implantierbare Prothese mit einer Rastprofilierung bekannt, die durch im Querschnitt halbkreisförmige Nuten an der Implantatoberfläche erzeugt wird. Die Nuten weisen keine Hinterschnitte auf, wodurch die Langzeitstabilität nach der Implantation nicht optimal ist.

Aufgabe der Erfindung ist es, eine Endoprothese der eingangs genannten Art derart weiterzubilden, daß nach der Implantation ein ausreichend fester Verbund zwischen Knochengewebe und der Endoprothese erreicht wird, die Herstellung der Oberflächenstruktur jedoch exakt reproduzierbar und einfach bleibt.

Die Vorteile der Erfindung liegen insbesondere darin, daß der Hinterschnitt - über die Länge der Nut gesehen - einen konstanten Querschnitt aufweist. Diese erfindungsgemäße Ausbildung der Nutenquerschnitte hat zur Folge, daß die erfindungsgemäße Endoprothese in einfacher Weise und reproduzierbar, beispielsweise mittels der Hochdruck-Wasserstrahl-Technik herstellbar ist. Bei der erfindungsgemäßen Verwendung der Hochdruck-Wasserstrahl-Technik zur Erzeugung der Nuten wird Gefügebeanspruchung und damit die Gefügeänderung an der Oberfläche reduziert, die Festigkeit der Endoprothese wird dadurch verbessert. Außerdem lassen sich die erfindungsgemäßen Endoprothesen mit den hinterschnittenen Nutenstrukturen exakt reproduzieren, so daß die Voraussetzung für die staatlichen Zulassungen gegeben sind. Die hinterschnittenen Nuten bewirken, daß das angrenzende Knochengewebe nach der Implantation durch die natürliche Regeneration des Knochengewebes in die Hinterschnitte hineinwächst und daher die Langzeitstabilität des Implantats/Knochenverbundes sichert. Zusammen mit dereinfachen Herstellbarkeit und genauen Reproduzierbarkeit und der durch die Hinterschnitte erreichbaren hohen Langzeitstabilität vereinigen die erfindungsgemäßen Endoprothesen Eigenschaften, deren gleichzeitige Realisierung bei bisher bekannten Endoprothesen nicht möglich waren.

Gemäß einer besonders bevorzugten Ausführungsform der Erfindung handelt es sich bei der Endoprothese insbesondere um ein Oberschenkelteil einer Hüftendoprothese, die einen Schaft zur Verankerung im Knochenhohlraum des Oberschenkelknochens aufweist. Die erfindungsgemäße Nutenstruktur wird bei einer derartigen Endoprothese bevorzugt im proximalen Bereich des Schaftes angeordnet, die Nuten verlaufen im wesentlichen quer oder senkrecht zur Schaftlängserstreckung an der Oberfläche des Schafts. Die Nuten sind bevorzugt lateral und/oder medial im proximalen Bereich über einen vergleichsweise geringen Umfangsabschnitt in Schaft-Umfangsrichtung angeordnet und ermöglichen nach dem Einwachsen des Knochengewebes in die Hinterschnitte einen festen Halt.

Gemäß einer besonders bevorzugten Ausführungsform der Erfindung verlaufen die Hinterschnitte der Nuten in Längsrichtung geradlinig. Die Hinterschnitte bilden - in Längsrichtung gesehen - also ein geradliniges uniformes Profil mit einem Querschnitt, der über die gesamte Länge der Hinterschnitte konstant ist. Diese Ausführungsform der Erfindung läßt sich in besonders einfacher Weise mittels eines Verfahrens verwirklichen, bei dem der Materialabtrag in geradliniger Arbeitsrichtung erfolgt. Da die Oberfläche der Endoprothese in der Regel an den Verlauf des Knochens angepaßt ist, in den die Endoprothese implantiert werden soll, besitzt diese Oberfläche in der Regel keine ebene Gestalt, sondern ist in verschiedenen Richtungen - von Ort zu Ort - unterschiedlich gekrümmt. Die geradlinige homogene Profilierung der Nutenhinterschnitte hat zur Folge, daß der Mittelpunkt der Hinterschnitte in der Längsrichtung einen jeweils von Ort zu Ort unterschiedlichen Abstand zur Prothesenoberfläche aufweist, wenn die Prothesenoberfläche beispielsweise dem Knochenhohlraum nachmodelliert ist und daher eine gekrümmte Oberfläche aufweist. Bevorzugt gehen daher die Hinterschnitte in einen Spalt über, der sich zur Oberfläche der Endoprothese hin erstreckt und die Nut spaltförmig an der Oberfläche der Endoprothese münden läßt.

Besonders bevorzugt lassen sich auch in Längsrichtung mehrere Nutenabschnitte aneinander ansetzen, deren Hinterschnitt-Profil oder-Querschnitt in Längsrichtung konstant ist, wobei die einzelnen Nutenabschnitte sich längs Geraden erstrecken, die gegeneinander unter einem vorgegebenen Winkel verlaufen. Auf diese Weise lassen sich die Nuten mit dem erfindungsgemäßen, in Längsrichtung homogenen und geradlinigen Profil auch über größere, gegebenenfalls stark gekrümmte Oberflächenbereiche der Endoprothese einformen.

Gemäßeiner bevorzugten Ausführungsform der Erfindung verlaufen mehrere Nuten parallel zueinander. In einer alternativen Ausführungsform läßt sich der Abstand der Nuten auch als eine Funktion des Ortes, an der sich die betreffenden Nuten an der Oberfläche der Endoprothese befinden, vorgeben.

Die Dichte der Nuten sowie die lichte Weite der Nuten läßt sich der Verteilung der Spongiosabälkchenstruktur (Trabekelstruktur) genau anpassen, wodurch das Einwachsen der Trabekel und damit die Verankerungsqualität der Endoprothese verbessert werden kann. Außerdem läßt sich die Dichte der Nuten an denjenigen Stellen der Endoprothese erhöhen, an denen eine besonders hohe Belastung der Knochen/Endoprothesen-Grenzfläche auftritt, wodurch die Festigkeit des Knochen/Endoprothesenverbundes an diesen Bereichen durch den zahlreichen Formschluß zwischen Knochengewebe und den hinterschnittenen Nuten erhöht wird.

Gemäß der Erfindung besitzt der Hinterschnitt der Nuten einen Kreisquerschnitt und ist als eine Durchgangsbohrung mit einer geraden Längsachse ausgebildet. Der Hinterschnitt der Nuten ist mittels eines Spaltes mit der Oberfläche der Endoprothese verbunden, die Nuten münden daher spaltförmig an der Oberfläche der Endoprothese. Die Stege zwischen den benachbarten Nuten besitzen bevorzugt eine sichtbare Oberfläche, welche von der Außenkontur der Endoprothese vorgegeben ist. Gemäß dieser Asuführungsform der Erfindung wird daher die Außenkontur der Endoprothese von der erfindungsgemäßen Nutenstruktur nicht verändert, die Paßgenauigkeit der Endoprothese relativ zu dem in den Knochen vorgearbeiteten Verankerungshohlraum wird nicht beeinträchtigt. Alternativ läßt sich jedoch die sichtbare Oberfläche der Stege mit einer zusätzlichen Makro- oder Mikrostruktur versehen, um die Festigkeit des Knochen-/Endoprothesenverbundes zu erhöhen.
Erfindungsgemäß werden die Hinterschnitte und/oder die Nuten teilweise oder ganz mittels eines Hochdruck-Wasserstrahls eingeformt. Bei dieser Herstellungsweise werden Kerben und Gefügeveränderungen weitgehend ausgeschaltet. Die erfindungsgemäßen Nutenstrukturen lassen sich insbesondere bei geradlinigem Verlauf der Hinterschnitte - mit einem durchgehenden Wasserstrahl erzeugen, der an der Austrittsstelle noch soviel kinetische Energie besitzt, daß Ausfransungen oder Kerben vermieden werden. Bei einer Kombination von spanabhebender Bearbeitung und Hochdruck-Wasserstrahl-Technik werden einerseits die bei spanabhebender Bearbeitung entstehenden Kerben geglättet, andererseits kann durch Zugabe von abrasiven Werkstoffen im Wasserstrahl eine mikrorauhe Oberfläche erzeugt werden, die das Anwachsen des Knochengewebes erleichtert. Das Wasserstrahl-Schneidverfahren setzt für harte Materialien abrasive Zusätze zum Wasserstrahl voraus. Diese können keramischen Ursprungs sein und bevorzugt eine definierte Korngröße oder Korngrößenverteilung aufweisen. Bei dieser Ausführungsform der Erfindung lassen sich mit der Herstellung der Nutenstruktur auch Mikrostrukturen an der Nutgrenzfläche erzeugen, welche das Einwachsen des Knochengewebes fördern und den Formschluß zwischen Endoprothese und Knochengewebe mikrostrukturell verbessern können. Besonders bevorzugt werden die Verfahrensparameter sowie die abrasiven Zusätze so eingestellt oder angewählt, daß die Hinterschnitte der Nuten bei ihrer Herstellung mit einer Oberflächenrauhigkeit zwischen 20 Mikrometer und 120 Mikrometer entstehen.

Vorteilhafte Weiterbildungen der Erfindung sind durch die Merkmale der Unteransprüche gekennzeichnet.

Im folgenden wird ein Ausführungsbeispiel der Erfindung anhand der Zeichnung näher erläutert. Es zeigen:
- Fig. 1: eine Seitenansicht des Oberschenkelteils einer Hüftgelenkendoprothese;
- Fig. 2: eine Ansicht der Endoprothese gemäß Fig. 1, von lateral gesehen;
- Fig. 3: einen Querschnitt längs der Linie III-III der Fig. 2; und
- Fig. 4: eine Seitenansicht der Struktur gemäß Fig. 3.

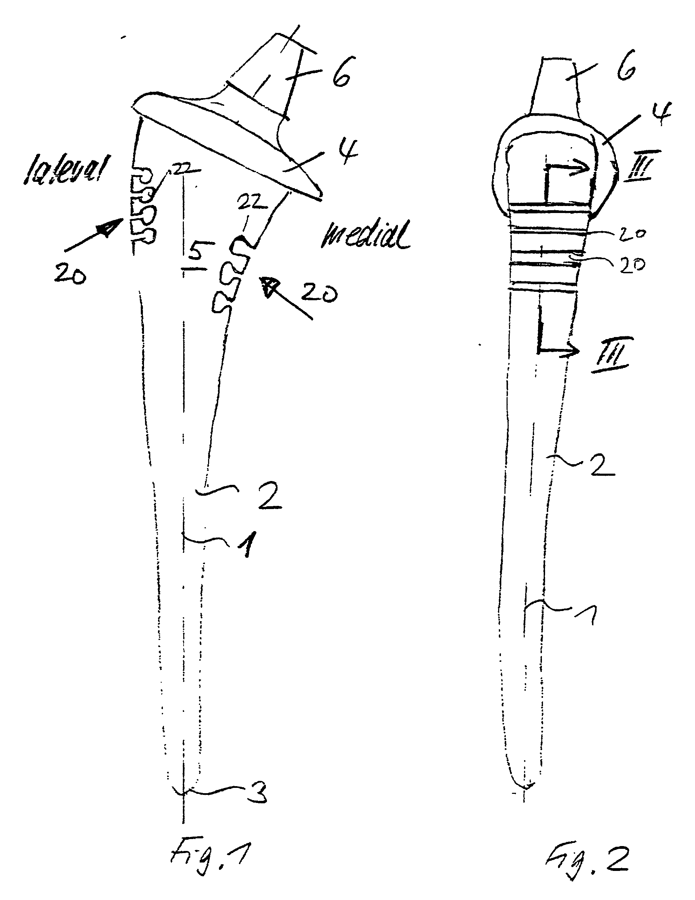

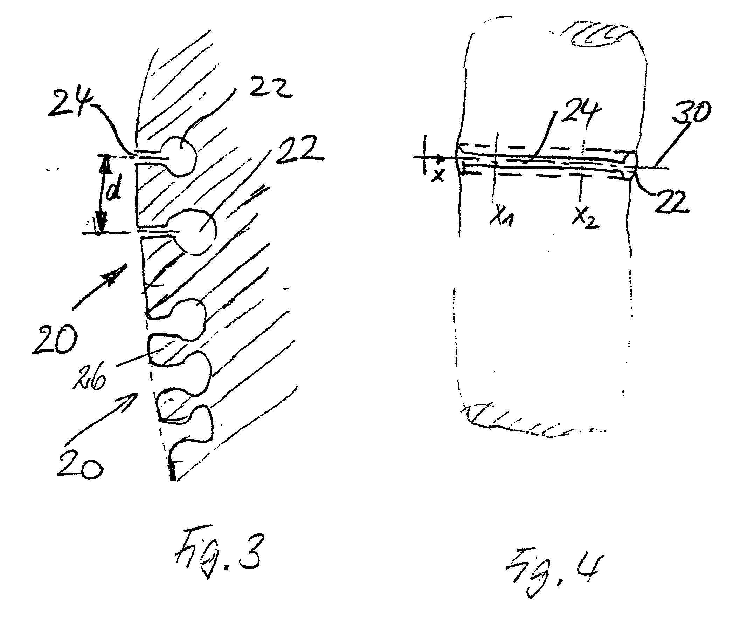

Die Fig. 1 bis 4 zeigen ein Oberschenkelteil einer Hüftgelenk-Endoprothese, von der Seite gesehen (Fig. 1), von lateral gesehen (Fig. 2) oder im Ausschnitt (Fig. 3 und 4). Die Endoprothese besitzt einen Schaft 2, der sich von einem distalen Ende 3 bis zu einem proximalen Bereich 5 erstreckt und proximal in einen Kragen 4 und einen anschließenden Steckkonus 6 übergeht, auf den ein Kugelkopf aufsteckbar ist. Im proximalen Bereich 5 des Schaftes sind lateral als auch medial mehrere Nuten 20 vorgesehen, die hinterschnitten sind, also Hinterschnitte 22 aufweisen.

Wie insbesondere den Fig. 3 und 4 entnommen werden kann, besitzen die Hinterschnitte über die Länge der Nuten 20 hin eine konstante Querschnittsform 22. Die Hinterschnitte erstrecken sich in Längsrichtung geradlinig. Die Hinterschnitte 22 gehen zur Oberfläche der Endoprothese hin in einen Spalt 24 über, der spaltförmig an der Oberfläche der Endoprothese mündet. Der Querschnitt einer sich in x-Richtung erstreckenden Nut, wobei x die Längsrichtung der Nut darstellt, vgl. Fig. 4, ist über die gesamte Längserstreckung der Nut konstant. Der Mittelpunkt der Hinterschnitt-Querschnitte - an den beliebigen Stellen x1, x2, ..., xn auf einer Geraden, so daß die Nut-Grenzfläche eine Zylinderfläche darstellt, die in Längsrichtung einen konstanten Querschnitt besitzt.

Wie insbesondere den Fig. 1 oder 3 entnehmbar ist, kann der Querschnitt der Hinterschnitte 22 von Nut zu Nut verschieden sein; benachbarte Nuten verlaufen - in der dargestellten Ausführungsform - parallel zueinander. Verschiedene Nuten können einen verschiedenen Abstand d voneinander haben.

Die Stege 26 zwischen benachbarten Nuten 20 besitzen eine sichtbare Oberfläche, welche im wesentlichen durch die Kontur der Oberfläche der Endoprothese vorgegeben ist.

## Patentansprüche

1. Zementfrei implantierbare Endoprothese aus Metall, mit mehreren an der Oberfläche eingeformten, im Querschnitt hinterschnittenen Nuten, wobei der Hinterschnitt (22) jeder Nut (20) über die Länge der Nut eine im wesentlichen konstanten Querschnitt aufweist, **dadurch gekennzeichnet, daß** der Hinterschnitt (22) der Nuten (20) einen Kreisquerschnitt aufweist.

2. Endoprothese, insbesondere Oberschenkelteil einer Hüftendoprothese nach einem der Ansprüche 1 bis 12, mit einem Schaft, **dadurch gekennzeichnet, daß** die Nuten (20) im wesentlichen quer oder senkrecht zur Schaft-Längserstreckung an der Oberfläche des Schafts eingeformt sind.

3. Endoprothese nach Anspruch 2, **dadurch gekennzeichnet, daß** die Nuten (20) im proximalen Bereich des Schaftes (2) angeordnet sind.

4. Endoprothese nach Anspruch 2 oder 3, **dadurch gekennzeichnet, daß** die Nuten (20) lateral und/oder medial im proximalen Bereich des Schaftes (2) angeordnet sind und abschnittsweise in Schaft-Umfangsrichtung verlaufen.

5. Endoprothese nach einem der vorstehenden Ansprüche, **dadurch gekennzeichnet, daß** der Hinterschnitt (22) jeder Nut sich geradlinig in Längsrichtung der Nut mit konstantem Querschnitt erstreckt.

6. Endoprothese nach einem der vorstehenden Ansprüche, **dadurch gekennzeichnet, daß** die Nuten (20) in ihrer Längsrichtung geradlinig verlaufen und einen im wesentlichen konstanten Querschnitt aufweisen.

7. Endoprothese nach einem der vorstehenden Ansprüche, **dadurch gekennzeichnet, daß** die Nuten (20) in einem vorgegebenen Bereich der Endoprothese parallel zueinander verlaufen.

8. Endoprothese nach einem der vorstehenden Ansprüche, **dadurch gekennzeichnet, daß** die Nuten (20) voneinander einen gleichbleibenden Abstand aufweisen.

9. Endoprothese nach einem der vorstehenden Ansprüche, **dadurch gekennzeichnet, daß** der Abstand der Nuten (20) eine Funktion des Ortes ist, an der sich die betreffenden Nuten (20) an der Oberfläche der Endoprothese befinden.

10. Endoprothese nach Anspruch 1, **dadurch gekennzeichnet, daß** der Hinterschnitt (22) der Nuten (20) als Durchgangsbohrung mit gerader Längsachse ausgebildet ist.

11. Endoprothese nach einem der vorstehenden Ansprüche, **dadurch gekennzeichnet, daß** die Stege (30) zwischen benachbarten Nuten (20) eine sichtbare Oberfläche aufweisen, welche von der Außenkontur der Endoprothese vorgegeben ist.

12. Endoprothese nach einem der vorstehenden Ansprüche, **dadurch gekennzeichnet, daß** die Rauhigkeit der Hinterschnitte (22) der Nuten (20) zwischen 20 Mikrometer und 120 Mikrometer beträgt.

13. Verfahren zur Herstellung einer Endoprothese nach einem der Ansprüche 1 bis 12, **dadurch gekennzeichnet, daß** die Nuten (20) mindestens teilweise mittels eines Hochdruck-Wasserstrahls erzeugt werden.

14. Verfahren nach Anspruch 13, **dadurch gekennzeichnet, daß** die Hinterschnitte (22) der Nuten (20) mittels eines Hochdruck-Wasserstrahls erzeugt werden.

15. Verfahren nach Anspruch 13 oder 14, **dadurch gekennzeichnet, daß** die Nutenstruktur mit einem geradlinig gerichteten Durchgangsstrahl im Hochdruck-Wasserstrahl-Verfahren erzeugt wird.

16. Verfahren nach einem der Ansprüche 13 bis 15, **dadurch gekennzeichnet, daß** dem Hochdruck-Wasserstrahl abrasive Zusätze zugesetzt werden.

## Claims

1. A metal endoprothesis which can be implanted without cement, with a plurality of grooves undercut in cross section formed on the surface, whereby an undercut (22) of each groove (20) has an essentially constant cross section over a length of the groove, **characterized in that** the undercut (22) of each groove (20) has a circular cross section.

2. The endoprothesis, in particular an upper leg part of a hip endoprothesis according to one of claims 1 through 12, with a shaft, **characterized in that** the grooves (20) are formed on the surface of the shaft essentially transverse or perpendicular to a longitudinal extension of the shaft.

3. The endoprothesis according to claim 2, **characterized in that** the grooves (20) are arranged approximately in a region of the shaft (2).

4. The endoprothesis according to claim 2 or 3, **characterized in that** the grooves (20) are arranged laterally and/or medially approximately in a region of the shaft (2) and run partially in a peripheral direction of the shaft.

5. The endoprothesis according to one of the preceding claims, **characterized in that** the undercut (22) of each groove extends in a straight line in a longitudinal direction of the groove with a constant cross section.

6. The endoprothesis according to one of the preceding claims, **characterized in that** the grooves (20) run in a longitudinal direction in a straight line and have an essentially constant cross section.

7. The endoprothesis according to one of the preceding claims, **characterized in that** the grooves (20) run parallel to one another in a predetermined region of the endoprothesis.

8. The endoprothesis according to one of the preceding claims, **characterized in that** the grooves (20) have a uniform distance from one another.

9. The endoprothesis according to one of the preceding claims, **characterized in that** the distance of the grooves (20) is a function of a location at which the respective grooves (20) are found on the surface of the endoprothesis.

10. The endoprothesis according to claim 1, **characterized in that** the undercut (22) of the groove (20) is formed as a through-hole with a straight longitudinal axis.

11. The endoprothesis according to one of the preceding claims, **characterized in that** cross pieces (30) between adjacent grooves (20) have a visible surface, which is predetermined from an outer contour of the endoprothesis.

12. The endoprothesis according to one of the preceding claims, **characterized in that** the undercuts (22) of the grooves (20) have a roughness between 20 micrometers and 120 micrometers.

13. A method for manufacturing an endoprothesis according to one of claims 1 through 12, **characterized in that** the grooves (20) are produced at least partially by means of a high-pressure water jet.

14. The method according to claim 13, **characterized in that** the undercuts (22) of the grooves (20) are produced by means of a high-pressure water jet.

15. The method according to claim 13 or 14, **characterized in that** the groove structure is produced with a through-going jet directed in a straight line in a high-pressure water jet method.

16. The method according to one of claims 13 through 15, **characterized in that** abrasive additives are added to the high-pressure water jet.

## Revendications

1. Endoprothèse métallique, implantable sans ciment, comportant plusieurs rainures formées en surface, découpées à l'arrière dans leur section transversale, la découpe arrière (22) de chaque rainure (20) présentant sur la longueur de la rainure une section transversale substantiellement constante, **caractérisée en ce que** la découpe arrière (22) des rainures (20) présente une section transversale circulaire.

2. Endoprothèse, notamment partie de la cuisse d'une prothèse de hanche selon une des revendications 1 à 12, comportant une tige, **caractérisée en ce que** les rainures (20) sont formées substantiellement transversalement ou verticalement par rapport à l'extension longitudinale de la tige à la surface de la tige.

3. Endoprothèse selon la revendication 2, **caractérisée en ce que** les rainures (20) sont disposées dans la zone proximale de la tige (2).

4. Endoprothèse selon la revendication 2 ou 3, **caractérisée en ce que** les rainures (20) sont disposées latéralement et/ou médialement dans la zone proximale de la tige (2) et s'étendent par sections dans le sens périphérique de la tige.

5. Endoprothèse selon une des revendications précédentes, **caractérisée en ce que** la découpe arrière (22) de chaque rainure s'étend en ligne droite dans le sens longitudinal de la rainure avec une section transversale constante.

6. Endoprothèse selon une des revendications précédentes, **caractérisée en ce que** les rainures (20) s'étendent en ligne droite dans leur sens longitudinal et présentent une section transversale substantiellement constante.

7. Endoprothèse selon une des revendications précédentes, **caractérisée en ce que** les rainures (20) s'étendent parallèlement les unes aux autres dans une zone prédéfinie de l'endoprothèse.

8. Endoprothèse selon une des revendications précédentes, **caractérisée en ce que** les rainures (20) se trouvent à une distance constante les unes des autres.

9. Endoprothèse selon une des revendications précédentes, **caractérisée en ce que** la distance entre les rainures (20) est fonction de l'endroit où se trouvent les rainures concernées (20) en surface de l'endoprothèse.

10. Endoprothèse selon la revendication 1, **caractérisée en ce que** la découpe arrière (22) des rainures (20) est. conçue sous forme d'un alésage continu avec un axe longitudinal droit.

11. Endoprothèse selon une des revendications précédentes, **caractérisée en ce que** les traverses (30) entre les rainures voisines (20) présentent une surface visible qui est définie par le contour extérieur de l'endoprothèse.

12. Endoprothèse selon une des revendications précédentes, **caractérisée en ce que** la rugosité des découpes arrière (22) des rainures (20) se situe entre 20 micromètres et 120 micromètres.

13. Procédé de fabrication d'une endoprothèse selon une des revendications 1 à 12, **caractérisé en ce que** les rainures (20) peuvent être réalisées au moins partiellement au moyen d'un jet d'eau à haute pression.

14. Procédé selon la revendication 13, **caractérisé en ce que** les découpes arrière (22) des rainures (20) sont réalisées au moyen d'un jet d'eau à haute pression.

15. Procédé selon la revendication 13 ou 14, **caractérisé en ce que** la structure des rainures est réalisée à l'aide d'un jet d'eau continu orienté en ligne droite suivant le procédé à jet d'eau à haute pression.

16. Procédé selon une des revendications 13 à 15, **caractérisé en ce que** des additifs abrasifs sont ajoutés au jet d'eau à haute pression.
